# EUROPEAN PATENT SPECIFICATION

(11) **EP 1 619 198 B1**
(45) Date of publication and mention of the grant of the patent: **14.10.2009**
(21) Application number: 05015316.2
(22) Date of filing: 14.07.2005
(51) Int. Cl.: C07D 501/12

(54) **Process for the preparation of amorphous cefuroxime axetil**
Verfahren zur Herstellung von amorphem Cefuroximaxetil
Procédé pour la préparation du cefuroxim acetil en forme amorphe

(30) Priority: 22.07.2004 CN 200410069398
(43) Date of publication of application: 25.01.2006
(73) Proprietor: Nanomaterials Technology Pte Ltd, Singapore 139944 (SG); Beijing University of Chemical Technology, Beijing 100029 (CN)
(72) Inventor: Chen, Jianfeng, Beijing, 100029 (CN); Zhong, Jie, Beijing, 100029 (CN); Shen, Zhigang, Beijing 100029 (CN); Zhang, Jiyado, Beijing, 100029 (CN)
(74) Representative: Teipel, Stephan

(56) References cited:
- WO-A-01/87893
- WO-A-02/16372
- WO-A-98/43980
- WO-A-99/65919
- US-A- 5 013 833
- US-A- 5 847 118
- CHEN JIAN-FENG ET AL: "Feasibility of preparing nanodrugs by high-gravity reactive precipitation." INTERNATIONAL JOURNAL OF PHARMACEUTICS. 9 JAN 2004, vol. 269, no. 1, 9 January 2004 (2004-01-09), pages 267-274, XP002350730 ISSN: 0378-5173
- DATABASE COMPENDEX [Online] ENGINEERING INFORMATION, INC., NEW YORK, NY, US; SHEN ZHI-GANG ET AL: "Properties of cephradine produced by high gravity technology" XP002350814 Database accession no. E2004188146338 & CHIN. PHARM. J. (CHINA); CHINESE PHARMACEUTICAL JOURNAL JANUARY 2004, vol. 39, no. 1, January 2004 (2004-01), pages 36-39,
- SASINOWSKA-MOTYL M ET AL: "ESTERS OF CEPHALOSPORINS. PART I. PERMEABILITY OF CEFUROXIME LIBERATED FROM ITS 1-ACETOXYETHYL ESTER THROUGH BIOLOGICAL MEMBRANES; INFLUENCE OF THE FORM AND SIZE OF THE ESTER PARTICLES" ACTA POLONIAE PHARMACEUTICA, POLISH PHARMACEUTICAL SOCIETY, WARZSAW, PL, vol. 52, no. 5, 1995, pages 391-395, XP000901626 ISSN: 0001-6837
- OSZCZAPOWICZ I ET AL: "ESTERS OF CEPHALOSPORINS. PART II. DIFFERENCES IN THE PROPERTIES OFVARIOUS FORMS OF THE 1-ACETOXYETHYL ESTER OF CEFUROXIME" ACTA POLONIAE PHARMACEUTICA, POLISH PHARMACEUTICAL SOCIETY, WARZSAW, PL, vol. 52, no. 5, 1995, pages 397-401, XP000901056 ISSN: 0001-6837

## Description

### FIELD OF THE INVENTION

This invention relates to a bioavailabile amorphous cefuroxime axetil and a preparation process therefore.

### BACKGROUND OF THE INVENTION

Cefuroxime axetil, i.e. (6R, 7R)-3-carbamoyloxymethyl-7-[(Z)-2-(fur-2-yl)-2-methoxyimino-acetylamido]-ceph-3-em-4-carboxylic acid 1-acetoxyethyl ester, is 1-acetoxyethyl ester of cefuroxime. As a high broad spectrum antibiotic against *cephalosporin,* cefuroxime axetil has a good antibiotic activity against both gram-positive and gram-negative microorganisms. The structure of the compound is as follows:

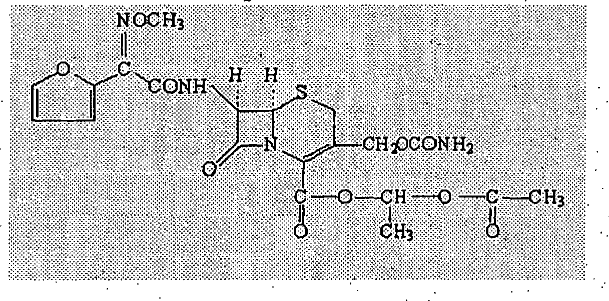

Cefuroxime axetil is present in two forms: crystalline and amorphous. GB 1,571,683 A1 disclose the process for the preparation of crystalline cefuroxime axetil. The crystalline cefuroxime axetil does not possess the necessary bioavailability characteristics associated with the amorphous form. It is known that orally administered cephalosporin (and medicaments in general) must be in a form of highly bioavailability. For this reason, commercially available cefuroxime axetil which is registered throughout the world is in a substantially amorphous form, since cefuroxime axetil in a substantially amorphous form has a higher bioavailability for oral administration than that in a crystalline form, as disclosed in U.S. Pat. No. 4,826,833 (Pat' 833).

Pat' 833 describes a process for the preparation of amorphous cefuroxime axetil, in which amorphous cefuroxime axetil is obtained by spray drying a solution of cefuroxime axetil of a crystalline form in an organic solvent. The current process for industrializing amorphous cefuroxime axetil is usually the spray drying techniques as described in Pat' 833. However, the disadvantages associated with those techniques are that, for example, the cost in equipments may be high, recycling the organic solvents may be difficult, and improper temperature control during drying process may affect the quality of the cefuroxime axetil.
US Patent No.5,0.13,833 discloses a process for preparing amorphous cefuroxime axetil by the spray drying techniques or by solvent precipitation. Neither an amorphous cefuroxime axetil particle with controllable particle size, nor an ultrafine or even nanosized amorphous cefuroxime axetil particle can be produced by those methods described in Patent 5,013,833.

J.-F. Chen, et al. in International Journal of Pharmaceutics 269 (2004) 267-274 describes a study the feasibility of producing nanoparticles of organic pharmaceuticals using a high-gravity reactive precipitation technique.

S. Zhi-Gang, et al. in Chin. Pharm. J. (China) 39 (2004) 36-39 describes the properties of cephadrine produced by high-gravity technology.

M. Sasinowska-Motyl, et al. in Acta Poloniae Pharmaceutica - Drug Research 52 (1995) 391-395 describes a correlation between the particles size of the amorphous cefuroxime and the concentrations of cefuroxime in vivo.

I. Oszczapowicz, et al. in Acta Poloniae Pharmaceutica - Drug Research 52 (1995) 397-401 discloses physico-chemical and microbiological properties of three different forms of the 1-acetoxyethyl ester of cefuroxime.

WO 99/65919 relates to a process for the preparation of cefuroxime axetil in an amorphous form.

WO 98/43980 discloses a process for preparing an amorphous cefuroxime axetil having a low melting point.

US 5,847,118 relates to a process for preparing pure amorphous cefuroxime axetil comprising the steps of dissolving crystalline cefuroxime axetil in a highly polar organic solvent and adding the resulting solution to water.

WO 92/16372 discloses a further method for preparation of amorphous cefuroxime axetil by dissolving cefuroxime axetil into an water-soluble organic acid and dropping the obtained solution in cooling water.

WO 01/87893 describes a process for the preparation of a pure amorphous form of cefuroxime axetil which involves dissolving crystalline cefuroxime axetil in acetic acid in recovering amorphous form of cefuroxime axetil form the solution by the addition of water.

None of those methods can be used to produce an amorphous cefuroxime axetil with both controllable particle size and ultrafine or even nanosized particle size. In addition, solvent precipitation which is carried out within the conventional stirred vessels does have some disadvantages, e.g. non-uniform mixing and local supersaturation, which may have an influence on the quality of the cefuroxime axetil power.
Consequently, this invention is directed to provide a process for the preparation of an ultrafine or nanosized amorphous cefuroxime axetil particle. Specifically, the invention provides a process for the preparation of an cefuroxime axetil particle having both controllable average particle size and narrow particle size distribution.

### SUMMARY OF THE INVENTION

Based on the techniques in the prior art, the inventor found that amorphous cefuroxime axetil can be obtained by, within a stirred reactor, mixing the solution and the antiasolvent via an atomizer for the solution; then precipitating and crystallizing.
Specifically, this invention provides a process for the preparation of ultrafine amorphous cefuroxime axetil, which comprises:
(1) providing a cefuroxime axetil solution and an appropriate antisolvent;
(2) spraying the solution via an atomizer into a stirred reactor in which the antisolvent is contained, thereby recrystallizing the cefuroxime axetil by means of antisolvent recrystallization; and
(3) collecting the slurry of the cefuroxime axetil obtained in step (2), filtering and drying the filtrate to obtain the ultrafine or nanosized cefuroxime axetil powders in an amorphous form.

During the process of precipitating and crystallizing according to the invention, the solution and the antisolvent are contacted each other sufficiently and uniformly, whereby achieving ultra-speed molecular micro-mixing, leading to overcome the limitations of non-uniform and insufficient mixing between the solution and the antisolvent, and avoid the typical local supersaturation associated with the known methods. Since the reactants are contacted and mixed sufficiently and uniformly according to the process of the invention, the precipitation time is decreased and the yield ratio is increased compared with those process in the prior art. Furthermore, less space is required in the invention, hence favoring mass production.

### BRIEF DESCRIPTION OF THE DRAWINGS

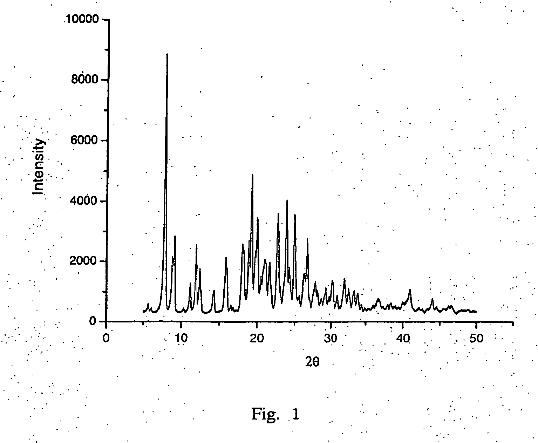

Fig. 1 shows X-ray diffractive spectrum of the crystalline cefuroxime axetil prepared by a conventional method.
Fig. 2 shows X-ray diffractive spectrum of the amorphous cefuroxime axetil prepared by the chloroform-isopropyl ether system according to the invention.

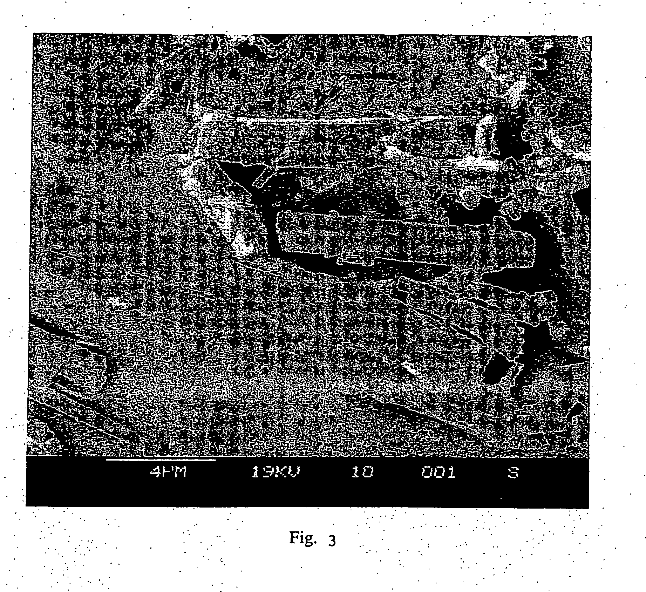

Fig. 3 shows an SEM(Scanning Electron Microscope) image of the cefuroxime axetil prepared by the conventional method.

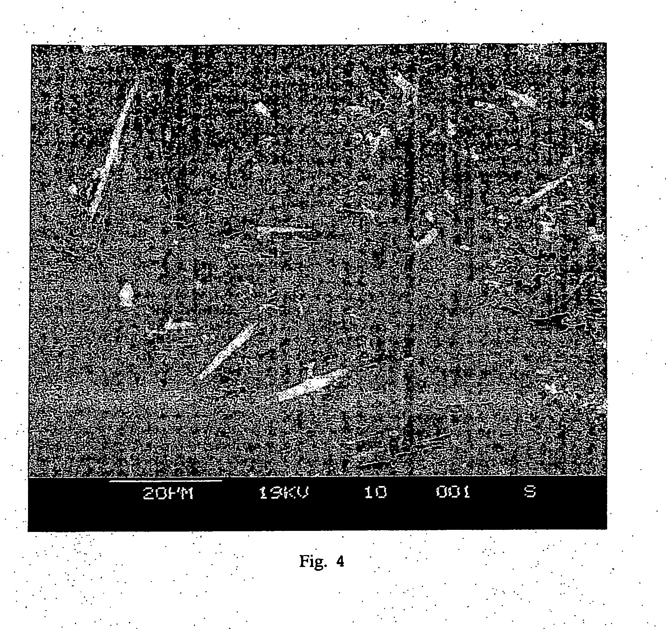

Fig. 4 shows an SEM image of the cefuroxime axetil prepared by a conventional method.

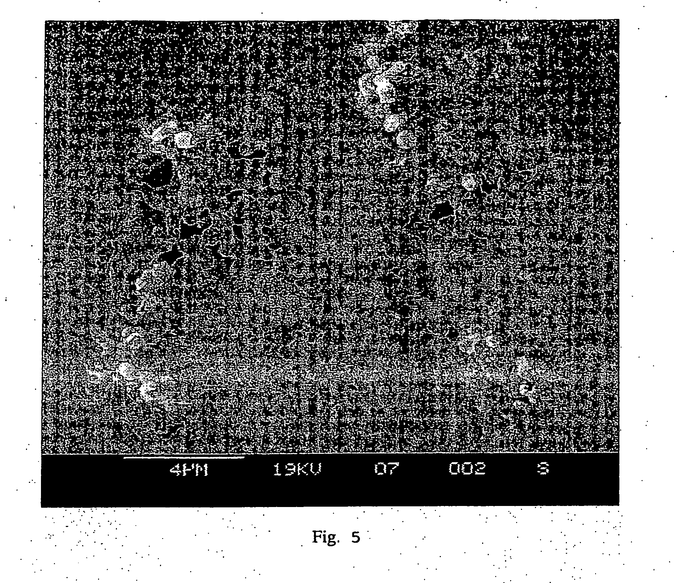

Fig. 5 shows an SEM image of the amorphous cefuroxime axetil prepared by the chloroform-isopropyl ether system according to the invention.

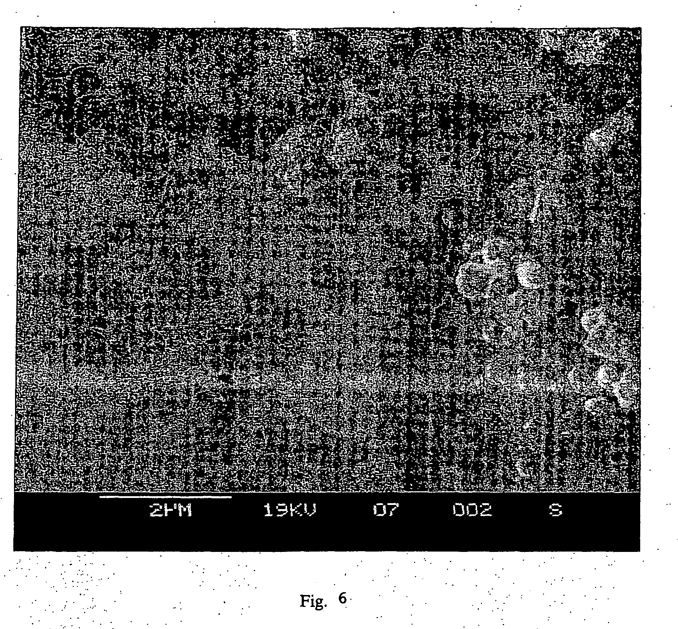

Fig. 6 shows an SEM image of the amorphous cefuroxime axetil prepared by the acetone-isopropyl ether system according to the invention.

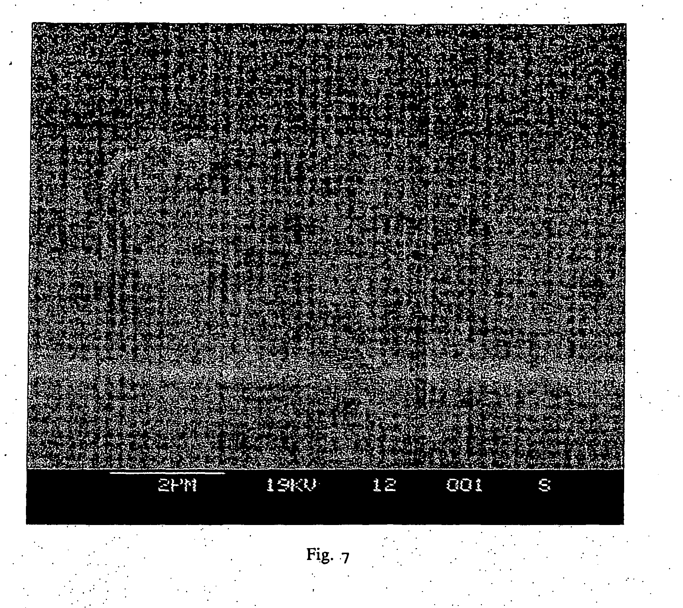

Fig. 7 shows an SEM image of the amorphous cefuroxime axetil prepared by the ethyl acetate-isopropyl ether system according to the invention.

### DETAILED DESCRIPTION OF THE INVENTION

The first aspect of the invention provides a process for the preparation of ultrafine cefuroxime axetil in an amorphous form, which comprises:
(1) providing a cefuroxime axetil solution and an appropriate antisolvent;
spraying the solution via an atomizer into a stirred reactor in which the antisolvent is contained, thereby recrystallizing the cefuroxime axetil by means of antisolvent recrystallization; and
(3) collecting the slurry of cefuroxime axetil obtained in step (2), filtering and drying the filtrate to obtain the ultrafine or nanosized cefuroxime axetil powders in an amorphous form.

By "crystallization" herein, it refers to the general "crystallization" and "recrystallization" processes, and it means the recovery of cefuroxime axetil particles, by means of crystallization or recrystallization, from any solution which contains cefuroxime axetil, including, for example, a solution of cefuroxime axetil in ethyl acetate, a solution of cefuroxime axetil in acetone, a solution of cefuroxime axetil in chloroform or a mixed solution of cefuroxime axetil in the above-mentioned solvents.

According to the invention, the antisolvent can be any solvent which is able to be mutually or partially miscible with the solution of cefuroxime axetil, and has a solubility as low as possible to the cefuroxime axetil. Preferably, the antisolvent comprises methyl tert-butyl ether, ethyl ether, isopropyl ether, n-hexane, water and the like.

The atomizer used in the invention can be any generally used atomizer in the art. For example, an atomizer comprising a stationary spray nozzle with a small orifice can be used, in which the spray nozzle is placed at the center of a high-speed packed rotor. Ultrafine atomized liquid droplets can be obtained by spraying the liquid feed out of the orifice of the spray nozzle with a certain speed, and then shearing and atomizing by the high-speed rotary packing layer.

The disadvantages associated with the conventional stirred vessel reactors are that the quality of the product is affected by local supersaturation, which is due to liquid feed can not be uniformly and rapidly mixed at each part of the reactor, upon entering into the reactor. In the present invention, the liquid feed can be atomized into ultrafine droplets immediately after the liquid is fed via the atomizer, and upon entering into the reactor, the liquid can be more rapidly and uniformly mixed with the solution (or the solvent) in the reactor, whereby avoiding any local supersaturation and thus improving the quality of the desired product. In addition, since the reactants are contacted and mixed sufficiently and uniformly, the precipitation time is decreased and the yield ratio is increased compared with those process in the prior art. Furthermore, particles having a more small and more uniform particle size can be obtained , due to the reaction (precipitating and crystallizing) of the atomized droplets in the reactor. Consequently, the size of the particles can be controlled by adjust the size of the orifice of the nozzle.

Alternatively, the solution of cefuroxime axetil and the antisolvent can be sprayed into the stirred reactor via different atomizers respectively.

In addition, it is possible to obtain ultrafine cefuroxime axetil particles with a controllable and uniform average particle size, particularly ultrafine cefuroxime axetil particles with a controllable average particle size and a narrow particle size distribution, by appropriately adjusting the operating parameters of the stirred vessel reactor such as the rotating speed of the stirred vessel and the size of the nozzle. The particles prepared according to the process of the invention can be used in the forms of tablet and capsule in the pharmaceutical field, since the particles do not have non-uniform particle size due to the non-uniform stirring associated with the techniques of the prior art. Furthermore, the particles due to its small and uniform particle size can be easily formulated into tablet and capsule and have a higher bioavailability.

In the method described above, the solution of cefuroxime axetil and the antisolvent simultaneously are sprayed into the stirred reactor via different atomizers.

The cefuroxime axetil solution used in the present invention includes any solution of cefuroxime axetil formed by dissolving the crystalline cefuroxime axetil as obtained in any appropriate solvent.

The commonly used solvents in the cefuroxime axetil solution comprises, for example, methanol, dichloromethane, chloroform, ethyl acetate, N,N-dimethyl formamide, formic acid, acetic acid, dioxane, acetone, dimethyl sulfoxide or the mixture thereof. The skilled in the art may choose any other solvents which can be used to dissolve cefuroxime axetil. In this invention, the term "dissolve" means that a clear solution is obtained by dissolving cefuroxime axetil in a solvent. The concentration of the cefuroxime axetil solution is not specially limited, as long as it can meet the requirement of dissolution.

The antisolvent used in the invention comprises methyl tert-butyl ether, isopropyl ether, ethyl ether, n-hexane, water and the like, preferably methyl tert-butyl ether, isopropyl ether, ethyl ether, and n-hexane.

According to the process of the present invention, cefuroxime axetil particles with the desired narrow particle size distribution can be obtained by adjusting the reaction conditions, e.g., the rotating speed of the, stirred reactor reaction temperature, and the flow rates of the solution and antisolvent. According to the invention, it provides cefuroxime axetil particles with a uniform particle size distribution, specially ultrafine or even nanosized cefuroxime axetil particles. Specifically, the present invention provides ultrafine cefuroxime axetil particles with a average particle size of usually less than 100 µm, preferably in the range between about 50 µm and about 20 nm, more preferably in the range between about 10µm and about 20 nm, and still preferably in the range between about 5 µm and about 20 nm.

Amorphous cefuroxime axetil particles with a narrow particle size distribution can be obtained by adjusting the reaction conditions, e.g., rotating speed, temperature and flow rate). The particles obtained by the method described in this invention differs from those obtained by the prior techniques in that the former has a narrow particle size distribution; that is, at least about 50%, preferably about 70%, and more preferably about 90% of the particles are present in the same order of particle size ranges, such as a particle size distribution of from 10 nm to 100 nm, from 200 nm and 500 nm, and from about 1µm to about 5 µm, and from about 5 µm to about 10 µm.

The cefuroxime axetil powders obtained by the method of the present invention have a purity of above 98%, and no less than 75% in terms of dry basis, as determined by HPLC (High Performance Liquid Chromatography).

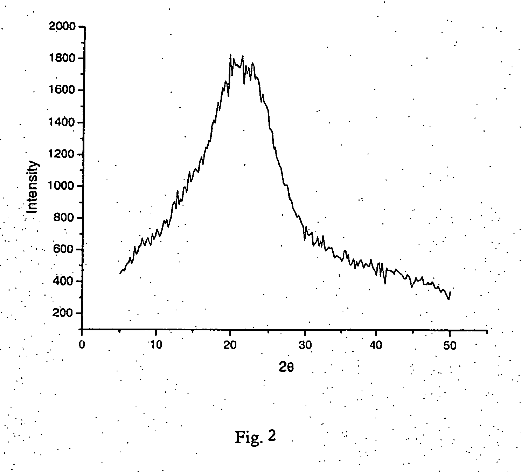

The amorphous cefuroxime axetil obtained by the method of the present invention have the X-ray diffractive spectrum shown in Fig.2, and the data of X-ray diffractive analysis is shown in Table 1.

**Table 1 The X-ray diffractive spectrum of amorphous cefuroxime axetil obtained in the present invention**

| Angle (2θ) | Intensity | Angle (2θ) | Intensity | Angle (2θ) | Intensity |
|---|---|---|---|---|---|
| 5 | 445 | 20.2 | 1804 | 35.2 | 548 |
| 5.2 | 460 | 20.4 | 1755 | 35.4 | 598 |
| 5.4 | 474 | 20.6 | 1767 | 35.6 | 596 |
| 5.6 | 463 | 20.8 | 1756 | 35.8 | 546 |
| 5.8 | 491 | 21 | 1742 | 36 | 572 |
| 6 | 512 | 21.2 | 1769 | 36.2 | 512 |
| 6.2 | 517 | 21.4 | 1821 | 36.4 | 503 |
| 6.4 | 553 | 21.6 | 1641 | 36.6 | 519 |
| 6.6 | 511 | 21.8 | 1761 | 36.8 | 545 |
| 6.8 | 540 | 22 | 1704 | 37 | 479 |
| 7 | 620 | 22.2 | 1746 | 37.2 | 530 |
| 7.2 | 570 | 22.4 | 1660 | 37.4 | 496 |
| 7.4 | 586 | 22.6 | 1777 | 37.6 | 530 |
| 7.6 | 625 | 22.8 | 1755 | 37.8 | 537 |
| 7.8 | 621 | 23 | 1669 | 38 | 514 |
| 8 | 674 | 23.2 | 1688 | 38.2 | 483 |
| 8.2 | 639 | 23.4 | 1670 | 38.4 | 515 |
| 8.4 | 625 | 23.6 | 1628 | 38.6 | 511 |
| 8.6 | 656 | 23.8 | 1529 | 38.8 | 498 |
| 8.8 | 676 | 24 | 1582 | 39 | 496 |
| 9 | 644 | 24.2 | 1532 | 39.2 | 542 |
| 9.2 | 622 | 24.4 | 1514 | 39.4 | 507 |
| 9.4 | 703 | 24.6 | 1499 | 39.6 | 483 |
| 9.6 | 663 | 24.8 | 1476 | 39.8 | 468 |
| 9.8 | 688 | 25 | 1367 | 40 | 437 |
| 10 | 730 | 25.2 | 1360 | 40.2 | 508 |
| 10.2 | 706 | 25.4 | 1341 | 40.4 | 518 |
| 10.4 | 681 | 25.6 | 1237 | 40.6 | 434 |
| 10.6 | 701 | 25.8 | 1251 | 40.8 | 518 |
| 10.8 | 741 | 26 | 1187 | 41 | 447 |
| 11 | 786 | 26.2 | 1164 | 41.2 | 388 |
| 11.2 | 762 | 26.4 | 1133 | 41.4 | 488 |
| 11.4 | 791 | 26.6 | 1121 | 41.6 | 470 |
| 11.6 | 741 | 26.8 | 1073 | 41.8 | 473 |
| 11.8 | 774 | 27 | 1018 | 42 | 466 |
| 12 | 806 | 27.2 | 1007 | 42.2 | 462 |
| 12.2 | 882 | 27.4 | 1013 | 42.4 | 442 |
| 12.4 | 906 | 27.6 | 978 | 42.6 | 482 |
| 12.6 | 857 | 27.8 | 914 | 42.8 | 469 |
| 12.8 | 973 | 28 | 918 | 43 | 461 |
| 13 | 885 | 28.2 | 867 | 43.2 | 454 |
| 13.2 | 923 | 28.4 | 849 | 43.4 | 448 |
| 13.4 | 905 | 28.6 | 831 | 43.6 | 442 |
| 13.6 | 989 | 28.8 | 807 | 43.8 | 419 |
| 13.8 | 998 | 29 | 822 | 44 | 418 |
| 14 | 959 | 29.2 | 791 | 44.2 | 445 |
| 14.2 | 1042 | 29.4 | 768 | 44.4 | 436 |
| 14.4 | 1096 | 29.6 | 747 | 44.6 | 406 |
| 14.6 | 1025 | 29.8 | 657 | 44.8 | 367 |
| 14.8 | 1047 | 30 | 748 | 45 | 390 |
| 15 | 1091 | 30.2 | 701 | 45.2 | 400 |
| 15.2 | 1112 | 30.4 | 692 | 45.4 | 429 |
| 15.4 | 1098 | 30.6 | 697 | 45.6 | 405 |
| 15.6 | 1085 | 30.8 | 716 | 45.8 | 408 |
| 15.8 | 1161 | 31 | 622 | 46 | 414 |
| 16 | 1187 | 31.2 | 639 | 46.2 | 433 |
| 16.2 | 1135 | 31.4 | 649 | 46.4 | 387 |
| 16.4 | 1207 | 31.6 | 683 | 46.6 | 389 |
| 16.6 | 1250 | 31.8 | 624 | 46.8 | 385 |
| 16.8 | 1237 | 32 | 657 | 47 | 398 |
| 17 | 1291 | 32.2 | 615 | 47.2 | 386 |
| 17.2 | 1281 | 32.4 | 692 | 47.4 | 386 |
| 17.4 | 1379 | 32.6 | 629 | 47.6 | 403 |
| 17.6 | 1420 | 32.8 | 592 | 47.8 | 387 |
| 17.8 | 1396 | 33 | 606 | 48 | 360 |
| 18 | 1463 | 33.2 | 619 | 48.2 | 361 |
| 18.2 | 1530 | 33.4 | 607 | 48.4 | 377 |
| 18.4 | 1476 | 33.6 | 616 | 48.6 | 349 |
| 18.6 | 1539 | 33.8 | 582 | 48.8 | 337 |
| 18.8 | 1620 | 34 | 550 | 49 | 357 |
| 19 | 1594 | 34.2 | 564 | 49.2 | 348 |
| 19.2 | 1662 | 34.4 | 561 | 49.4 | 331 |
| 19.4 | 1639 | 34.6 | 555 | 49.6 | 313 |
| 19.6 | 1562 | 34.8 | 549 | 49.8 | 290 |
| 19.8 | 1830 | 35 | 528 | 50 | 342 |
| 20 | 1694 | | | | |

The process of the present invention and the characteristics, features and advantages of the cefuroxime axetil particles obtained by the present process will be appropriated by those skilled in the art, with reference to the attached drawings and the following specific examples.

The invention will be further illustrated with reference to the following examples, while those examples are only illustrative of the invention and not intended to limit the scope thereof.

The raw materials of cefuroxime axetil in its crystalline form are used in the examples, which can be commercially available, or can be prepared by the methods disclosed in GB 1,571,683 A1 or Chinese patent applications No. 01814420.9(with the publication number of 1447812A), which are all incorporated herein by reference.

### Examples

### Example 1

2000 ml of the solution of cefuroxime axetil in chloroform with a concentration of 0.1 g/ml was formulated. 10 liters of ethyl ether was added into the stirred vessel as the antisolvent. The solution of cefuroxime axetil in chloroform was fed through the inlet, and then atomized by the orifice, followed by the rapid mixing with the antisolvent with a stirring speed of 1000 rpm. A white precipitate of cefuroxime axetil was obtained. The resultant slurry was immediately filtrated and the filtrate was washed and dried, to produce the ultrafine amorphous cefuroxime axetil particles. The cefuroxime axetil particles had an average particle size of about 400 nm, and at least 90% of the particles had a particle size ranging from 300 nm to 400 nm.

### Example 2

1000 ml of the solution of cefuroxime axetil in formic acid with a concentration of 0.3 g/ml was formulated. 15 liters of water was added into the stirred vessel as the antisolvent. The solution of cefuroxime axetil in formic acid was fed through the inlet, and then atomized by the orifice, followed by the rapid mixing with the antisolvent with a stirring speed of 1000 rpm. A white precipitate of cefuroxime axetil was obtained. The resultant mixture was immediately filtrated, and the filtrate was washed and dried under vacuum at 60 °C, to produce the ultrafine amorphous cefuroxime axetil particles. The cefuroxime axetil particles had an average particle size of about 1 µm. In the operation, the ratio of flow rates between the solution of cefuroxime axetil and water was about 1:15.

### Example 3

1000 ml of the solution of cefuroxime axetil in acetone with a concentration of 0.1 g/ml was formulated. 15 liters of isopropyl ether at a temperature of 5 °C was added into the stirred vessel as the antisolvent. The solution of cefuroxime axetil in acetone was added into the isopropyl ether via the orifice of the atomizer, and mixed rapidly with isopropyl ether. A white precipitate of cefuroxime axetil was immediately obtained. The resultant slurry of cefuroxime axetil was immediately filtrated and the filtrate was washed and dried under vacuum at 60 °C, to produce the ultrafine amorphous cefuroxime axetil particles. The cefuroxime axetil particles had an average particle size of about 500 nm. In the operation, the ratio of flow rates between the solution of cefuroxime axetil and isopropyl ether water was about 1:15, with the rotating speed of the rotary bed at 1000 rpm.

### Conclusion

It can be seen from the above description in combination with the specific result data and the drawings that a ultrafine cefuroxime axetil powder can be prepared by precipitating and crystallizing with the help of a antisolvent within the stirred vessel. The resultant powder are significantly smaller and more inform than those particles in the conventional crystalline form. In addition, the average particle size of the particles can be controlled , if desired. The particles have a narrow and uniform particle size distribution, leading to unexpected effects. In particular, the requirement of pulverization treatment under special condition is avoided.

In comparison to the standards of '2000 Chinese pharmacopeias'(the second edition), all the results of the ultrafine cefuroxime axetil in an amorphous form according to the invention conformed perfectly. SEM images show that the particle size was approximately 300 to 500nm. Also, the amorphous form of cefuroxime axetil had a higher bioavailability than its crystalline form. Therefore, the present invention provides a cefuroxime axetil particle with a controllable average particle size and narrow particle distribution, particularly an ultrafine or even nanosized amorphous cefuroxime axetil particle, more particularly an ultrafine or even nanosized cefuroxime axetil particle with a controllable average particle size and narrow particle distribution.

In preparing amorphous cefuroxime axetil particles by means of the antisolvent precipitation in the stirred vessel reactor, the liquid was immediately atomized into very small droplets due to a special feeding inlet. The solution and antisolvent were mixed rapidly under high-speed stirring, to facilitate a uniform concentration distribution within the whole stirred vessel, thus avoiding the presence of local non-uniformity and supersaturation, and also agglomeration and adhesive bonding of cefuroxime axetil particles. Consequently, the quality and grade of the amorphous cefuroxime axetil was improved. The reaction could also be carried out with appropriately increasing the temperature or without the use of low temperature. Moreover, the yield ratio was enhanced drastically.

The ultrafine amorphous cefuroxime axetil prepared by the method described in this invention has many advantages, such as small particle size, uniformity, narrow particle size distribution, and good fluidity) and so on. In medical applications, it brings about unexpected effects on , for example, bioavailability and solubility over the prior art.

## Claims

1. A process for the preparation of ultrafine or nanosized powder of amorphous cefuroxime axetil, comprising:
(1) providing a cefuroxime axetil solution and a suitable antisolvent;
(2) spaying the cefuroxime axetil solution via an atomizer into a stirred reactor which contains the antisolvent, thereby recrystallizing the cefuroxime axetil by means of antisolvent recrystallization; and
(3) collecting the slurry of cefuroxime axetil obtained in step (2), then filtering and drying the filtrate to obtain the ultrafine or nanosized amorphous cefuroxime axetil powder.

2. The process of claim 1 wherein the cefuroxime axetil solution is formed by dissolving cefuroxime axetil in a solvent selected from the group consisting of methanol, dichloromethane, chloroform, acetone, ethyl acetate, formic acid, acetic acid, dioxane, dimethyl sulfoxide, N,N-dimethyl formamide or the mixture thereof.

3. The process of any one of the preceding claims wherein the antisolvent comprises isopropyl ether, methyl tert-butyl ether, ethyl ether, n-hexane, water or the mixture thereof.

4. The process of any one of the preceding claims wherein the cefuroxime axetil solution comprises a solution of cefuroxime axetil in ethyl acetate, a solution of cefuroxime axetil in acetone, a solution of cefuroxime axetil in chloroform, preferably a solution of cefuroxime axetil in acetone, and a solution of cefuroxime axetil in ethyl acetate or the mixture thereof.

5. The process of any one of the preceding claims wherein the stirred reactor having a stirred speed ranging from about 50 rpm to about 10,000 rpm.

6. The process of any one of the preceding claims wherein the reaction temperature is between -25°C and 70°C, preferably between -10°C and 50°C.

7. The process of any one of the preceding claims wherein the ratio of the solution to the antisolvent is between 1:5 and 1:50, preferably between 1:10 and 1:30.

8. The process of any one of the preceding claims wherein the cefuroxime axetil have a particle size of from 20 nm to 30 µm.

## Patentansprüche

1. Verfahren zur Herstellung eines Ultrafein- oder Nanopulvers aus amorphem Cefuroxim-Axetil, umfassend:
(1) das Bereitstellen einer Cefuroxim-Axetil-Lösung und eines geeigneten Antisolvents;
(2) das Sprühen der Cefuroxim-Axetil-Lösung mit einem Zerstäuber in einen Rührreaktor, der das Antisolvent enthält, wodurch das Cefuroxim-Axetil mittels Antisolvent-Umkristallisation umkristallisiert wird; und
(3) das Sammeln der in Schritt (2) erhaltenen Aufschlämmung von Cefuroxim-Axetil, dann das Filtern und Trocknen des Filtrats, wodurch das amorphe Ultrafein- oder Nano-Cefuroxim-Axetil-Pulver erhalten wird.

2. Verfahren nach Anspruch 1, wobei die Cefuroxim-Axetil-Lösung durch Auflösen von Cefuroxim-Axetil in einem Lösungsmittel, ausgewählt aus der Gruppe, bestehend aus Methanol, Dichlormethan, Chloroform, Aceton, Ethylacetat, Ameisensäure, Essigsäure, Dioxan, Dimethylsulfoxid, N,N-Dimethylformamid oder einem Gemisch.davon, gebildet wird.

3. Verfahren nach einem der vorstehenden Ansprüche, wobei das Antisolvent Isopropylether, Methyl-tert-butylether, Ethylether, n-Hexan, Wasser oder ein Gemisch davon umfasst.

4. Verfahren nach einem der vorstehenden Ansprüche, wobei die Cefuroxim-Axetil-Lösung eine Lösung von Cefuroxim-Axetil in Ethylacetat, eine Lösung von Cefuroxim-Axetil in Aceton, eine Lösung von Cefuroxim-Axetil in Chloroform, bevorzugt eine Lösung von Cefuroxim-Axetil in Aceton, und eine Lösung von Cefuroxim-Axetil in Ethylacetat oder ein Gemisch davon umfasst.

5. Verfahren nach einem der vorstehenden Ansprüche, wobei der Rührreaktor eine Rührgeschwindigkeit von etwa 50 U/min bis etwa 10.000 U/min hat.

6. Verfahren nach einem der vorstehenden Ansprüche, wobei die Reaktionstemperatur zwischen -25 °C und 70 °C, bevorzugt zwischen -10 °C und 50 °C, liegt.

7. Verfahren nach einem der vorstehenden Ansprüche, wobei das Verhältnis der Lösung zu dem Antisolvent zwischen 1 : 5 und 1: 50, bevorzugt zwischen 1:10 und 1 : 30, liegt.

8. Verfahren nach einem der vorstehenden Ansprüche, wobei das Cefuroxim-Axetil eine Teilchengröße von 20 nm bis 30 µm hat.

## Revendications

1. Procédé pour la préparation de poudre ultrafine ou de dimension nanométrique de céfuroxime axetil amorphe, comprenant les opérations consistant à :
(1) se procurer une solution de céfuroxime axetil et un anti-solvant approprié ;
(2) pulvériser la solution de céfuroxime axetil par l'intermédiaire d'un atomiseur dans un réacteur agité qui contient l'anti-solvant, permettant ainsi de recristalliser le céfuroxime axetil au moyen d'une recristallisation par ajout d'anti-solvant ; et
(3) recueillir la bouillie de céfuroxime axetil obtenue à l'étape (2), puis filtrer et sécher le filtrat pour obtenir la poudre de céfuroxime axetil amorphe ultrafine ou de dimension nanométrique.

2. Procédé selon la revendication 1, dans lequel la solution de céfuroxime axetil est formée par dissolution de céfuroxime axetil dans un solvant choisi dans le groupe constitué par le méthanol, le dichlorométhane, le chloroforme, l'acétone, l'acétate d'éthyle, l'acide formique, l'acide acétique, le dioxane, le diméthyl sulfoxide, le N,N-diméthyl formamide ou leur mélange.

3. Procédé selon l'une quelconque des revendications précédentes, dans lequel l'anti-solvant comprend l'isopropyl éther, le méthyl tert-butyl éther, l'éthyl éther, le n-hexane, l'eau ou leur mélange.

4. Procédé selon l'une quelconque des revendications précédentes, dans lequel la solution de céfuroxime axetil comprend une solution de céfuroxime axetil dans l'acétate d'éthyle, une solution de céfuroxime axetil dans l'acétone, une solution de céfuroxime axetil dans le chloroforme, de préférence une solution de céfuroxime axetil dans l'acétone, et une solution de céfuroxime axetil dans l'acétate d'éthyle ou leur mélange.

5. Procédé selon l'une quelconque des revendications précédentes, dans lequel le réacteur agité a une vitesse d'agitation se situant dans la plage allant d'environ 50 tpm à environ 10000 tpm.

6. Procédé selon l'une quelconque des revendications précédentes, dans lequel la température de réaction se situe entre -25°C et 70°C, de préférence entre -10°C et 50°C.

7. Procédé selon l'une quelconque des revendications précédentes, dans lequel le rapport de la solution à l'anti-solvant se situe entre 1:5 et 1:50, de préférence entre 1:10 et 1:30.

8. Procédé selon l'une quelconque des revendications précédentes, dans lequel le céfuroxime axetil a une dimension de particule de 20 nm à 30 µm.
